# EUROPEAN PATENT APPLICATION

(11) **EP 4 840 845 A1**
(43) Date of publication of application: **18.11.2026**
(21) Application number: 25194985.5
(22) Date of filing: 08.08.2025
(51) Int. Cl.: C07C 303/02, C07C 309/15

(54) **A PROCESS FOR PREPARATION OF 2-ACRYLAMIDO-2-METHYLPROPANESULPHONIC ACID**

(30) Priority: 13.05.2025 IN 202541046103
(71) Applicant: Thirumalai Chemicals Ltd., Chennai Tamil Nadu 600015 (IN)
(72) Inventor: RANGASWAMY, Parthasarathy, 632403 Ranipet (IN); GOPAL IYER, Subramanian, 632403 Ranipet (IN); GURUNATHAN, Satheeshkumar, 632403 Ranipet (IN)
(74) Representative: Hautier IP - MC/EP

(57) **Abstract**

The present disclosure relates to a process for preparing 2-acrylamido-2-methylpropanesulphonic acid (AMPS). The process involves feeding a first reactor with acrylonitrile, isobutene, and adding a sulphonating agent in two parts, under stirring. The first reactor is then re-fed with acrylonitrile, the sulphonating agent, and isobutene to obtain a mixture. The mixture overflows into a second reactor to form a slurry, which is then processed to obtain AMPS. The obtained AMPS is then subjected to purification. The process is carried out in anhydrous conditions, and acrylonitrile and isobutene are maintained in a mole ratio range of 4:1 to 7:1.

## Description

### FIELD OF INVENTION

The present disclosure generally relates to the field of chemical synthesis, specifically on preparation of sulphonic acid derivatives. In particular, the present disclosure relates to a process of preparation of 2-acrylamido-2-methylpropanesulphonic acid (AMPS), a compound widely used in the various fields and for the production of water-soluble polymers.

### BACKGROUND OF THE INVENTION

2-acrylamido-2-methylpropanesulphonic acid (AMPS) is a sulphonic acid acrylic monomer widely used in various industries due to its high reactivity and versatility. It is commonly used in the production of polymers and copolymers, which have applications in areas such as water treatment, oil recovery, personal care products, and coatings. AMPS is also used as a reactive emulsifier in the production of latexes and as a superplasticizer in the production of concrete.

The preparation of AMPS typically involves the reaction of acrylonitrile with a sulphonating agent, such as oleum, in the presence of a gaseous alkene, such as isobutene. The reaction is typically carried out in a reactor, which can be operated in a batch, semi-continuous, or continuous mode. The reaction conditions, including the temperature, pressure, and stirring speed, as well as the molar ratios of the reactants, can greatly influence the yield and purity of the resulting AMPS. However, the existing processes for the preparation of AMPS face challenges in aspects of purity and yield along with consumption of higher amounts of various reagents.

Therefore, there is a dire need in the art to develop a convenient yet efficient process for preparation of AMPS with high yield and purity.

### SUMMARY OF THE INVENTION

In an aspect of the present disclosure, there is provided a process for preparation of 2-acrylamido-2-methylpropanesulphonic acid, the process comprising: **a.** feeding a first reactor with acrylonitrile, isobutene, and adding a first part of a sulphonating agent under stirring, followed by addition of a second part of the sulphonating agent; **b.** refeeding the first reactor with acrylonitrile, the sulphonating agent and isobutene in an amount same as in the first step, under stirring to obtain a mixture; **c.** allowing the mixture to overflow to a second reactor to obtain a slurry; and **d.** processing the slurry to obtain 2-acrylamido-2-methylpropanesulphonic acid, wherein acrylonitrile and isobutene are in a mole ratio range of 4:1 to 7:1.

In another aspect of the present disclosure, there is provided a composition comprising: a) 2-acrylamido-2-methylpropanesulphonic acid; b) 2-methyl-2-propenyl-1-sulphonic acid; and c) 2-methylidene-1,3-propylenedisulphonic acid.

These and other features, aspects, and advantages of the present subject matter will be better understood with reference to the following description and appended claims. This summary is provided to introduce a selection of concepts in a simplified form. This summary is not intended to identify key features or essential features of the claimed subject matter, nor is it intended to be used to limit the scope of the claimed subject matter.

### BRIEF DESCRIPTION OF THE DRAWINGS

In order that the disclosure may be readily understood and put into practical effect, reference will now be made to exemplary embodiments as illustrated with reference to the accompanying figures. The figures together with a detailed description below, are incorporated in and form part of the specification, and serve to further illustrate the embodiments and explain various principles and advantages, in accordance with the present disclosure.

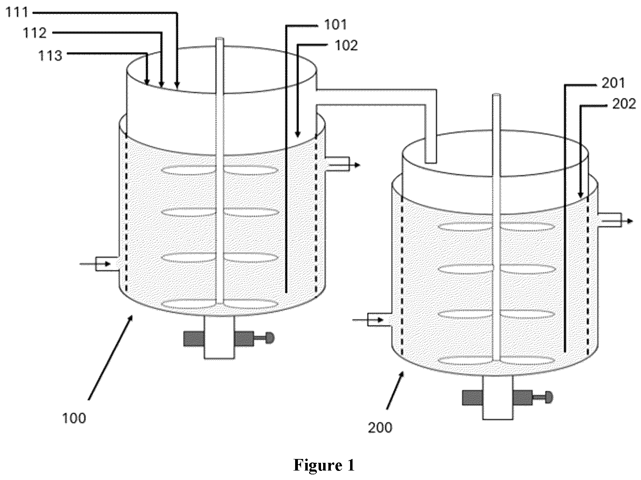

Figure 1 depicts the schematic representation of the first reactor (100) and second reactor (200) with the circulator arrangement, wherein the reactants acrylonitrile (111), isobutene (112), and oleum (113) are fed into the first reactor; and first reactor and second reactor have temperature indicators (101 and 201) and nitrogen blanketing pipes (102 and 202) attached, in accordance with an embodiment of the present disclosure.

### DETAILED DESCRIPTION OF THE INVENTION

The following description sets forth exemplary aspects of the present disclosure. It should be recognized, however, that such a description is not intended as a limitation on the scope of the present disclosure. Rather, the description also encompasses combinations and modifications to those exemplary aspects described herein. Those skilled in the art will be aware that the present disclosure is subject to variations and modifications other than those specifically described. It is to be understood that the present disclosure includes all such variations and modifications. The disclosure also includes all such steps, features, compositions, and compounds referred to or indicated in this specification, individually or collectively, and any and all combinations of any or more of such steps or features.

### Definitions

For convenience, before further description of the present disclosure, certain terms employed in the specification, and examples are delineated here. These definitions should be read in the light of the remainder of the disclosure and understood as by a person of skill in the art. The terms used herein have the meanings recognized and known to those of skill in the art, however, for convenience and completeness, particular terms and their meanings are set forth below.

The articles "a", "an" and "the" are used to refer to one or to more than one (i.e., to at least one) of the grammatical object of the article.

The terms "comprise" and "comprising" are used in the inclusive, open sense, meaning that additional elements may be included. It is not intended to be construed as "consists of only".

Throughout this specification, unless the context requires otherwise the word "comprise", and variations such as "comprises" and "comprising", will be understood to imply the inclusion of a stated element or step or group of element or steps but not the exclusion of any other element or step or group of element or steps.

The term "including" is used to mean "including but not limited to". "Including" and "including but not limited to" are used interchangeably.

The term "mixture" refers to combination of compounds obtained as result of feeding and/or refeeding acrylonitrile, and a sulphonating agent such as oleum into a reactor, wherein the mixture does not have residual water content.

The term "sulphonating agent" refers to one or more sulphur containing reagents, which when contacted with a compound facilitates introduction of sulphur or sulphur containing moieties into the compound. In an aspect of the present disclosure, the sulphonating agent comprises sulphuric acid with 1 to 30% (w/w) sulphur trioxide, with respect to total weight of the sulphonating agent.

The term "oleum" refers to a solution comprising a particular weight percentage of sulphur trioxide in anhydrous sulphuric acid. In an aspect of the present disclosure, a first part of the sulphonating agent has sulphur trioxide in an amount ranging from 20 to 30% (w/w) with respect to total weight of the first part of the sulphonating agent; and a second part of the sulphonating agent has sulphur trioxide in an amount ranging from 1 to 5% (w/w) with respect to total weight of the second part of the sulphonating agent.

The term "feeding" and "refeeding" refers to the charging of the first reactor with reactants, namely, acrylonitrile, the sulphonating agent and isobutene.

The term "continuous process" refers to the process by which at least one reactor chamber is continuously or intermittently fed with reactants to facilitate the continuous flow of products from the reactor chamber.

The term "semi- continuous process" as used herein refers to the process wherein the flow of products from a reactor chamber wherein the reactants are contacted, is in a discontinuous manner.

The term "batch process" refers to a manufacturing process involving producing a certain number of products in a given quantity at a time. In batch processes, the specific amounts of products are obtained after one reaction is completely over and the next set of reactants are reloaded into the reactor chamber.

The term "reactor" refers to a reacting chamber wherein a reaction between two or more reactants takes place by controlling the necessary physical parameters such as temperature, pressure, flow rate, weight or volume of reactants etc. In an aspect of the present disclosure, the process is carried out in two reactors namely a first reactor (100) and a second reactor (200). In another aspect of the present disclosure, the reactor, which is made of a metal or metal alloy, such as nickel, chromium, molybdenum, or combinations thereof, preferably the reactors are made of Inconel 825 and/or Hastelloy C 22.

The term "circulator arrangement" refers to the arrangement of the first reactor and second reactor using a pump, a valve, a conduit, or combinations thereof. The circulator arrangement of the reactor facilitates thorough mixing of the reactants. The pump is configured to pump the reactants and the reaction mixtures from one part of the process to another. The valve is configured to control the flow rate of the reactants and the reaction mixtures throughout the process. The conduit is configured to provide a flow path for the reactants and the reaction mixtures throughout the process. In yet another embodiment of the present disclosure, the circulator arrangement is used to facilitate the overflow of the second mixture from the first reactor to the second reactor. The circulator arrangement is configured to ensure a continuous flow of the second mixture from the first reactor to the second reactor which helps to ensure a thorough mixing and reaction between the reactants which are acrylonitrile, the sulphonating agent, and the isobutene. Figure 1 depicts the schematic representation of the first reactor (100) and second reactor (200) with the circulator arrangement. The Figure 1 shows feeding the reactants acrylonitrile (111), isobutene (112), and oleum (113) into the first reactor. Furthermore, the Figure 1 shows the first reactor and second reactor have temperature indicators (101 and 201) and nitrogen blanketing pipes (102 and 202) attached.

The term "residence time" is the operating volume of a reactor divided by the flow rate of precursors. In an aspect of the present disclosure, the specific residence times for the acrylonitrile, isobutene, and the sulphonating agent is selected to optimize the reaction conditions and to ensure a high yield and purity of 2-acrylamido-2-methylpropanesulphonic acid. In another aspect of the present disclosure, acrylonitrile has a residence time in a range of 3 to 5 hours, isobutene has a residence time in a range of 15 to 21 hours, and the sulphonating agent has a residence time in a range of 27 to 37 hours.

The term "recovery%" or "recovered" as used herein refers to amount of a material retrieved after subjecting crude of the material to a particular process. For the purpose of the present disclosure, "recovery %" or "recovered" refers to the amount of 2-acrylamido-2-methylpropanesulphonic acid (AMPS) recovered from crude product after purification process.

The term "purification" refers to the process of purifying or separating out a compound from a mixture of two or more compounds by techniques such as distillation, chromatography, crystallization, etc. In an aspect of the present disclosure, the AMPS prepared by the process as disclosed herein is subjected to purification wherein obtained crude product is distilled and crystallized using an organic solvent like acetic acid followed by washing and separating.

Ratios, concentrations, amounts, and other numerical data may be presented herein in a range format. It is to be understood that such range format is used merely for convenience and brevity and should be interpreted flexibly to include not only the numerical values explicitly recited as the limits of the range, but also to include all the individual numerical values or sub-ranges encompassed within that range as if each numerical value and sub-range is explicitly recited. For example, a temperature range of 0 °C to 20 °C should be interpreted to include not only the explicitly recited limits of 0 °C to 20 °C, but also to include subranges, such as 1 °C to 10 °C, 5 °C to 20 °C, 15 °C to 20°C and so forth, as well as individual amounts, including fractional amounts, within the specified ranges, such as 9 °C, 10.5 °C, 11.6 °C, 15.5 °C and 19.9 °C, for example.

Unless defined otherwise, all technical and scientific terms used herein have the same meaning as commonly understood by one of ordinary skill in the art to which this disclosure belongs. Although any methods and materials similar or equivalent to those described herein can be used in the practice or testing of the disclosure, the preferred methods and materials are now described. All publications mentioned herein are incorporated herein by reference.

As discussed in the background, there is a need to develop a process of preparation of 2-acrylamido-2-methylpropanesulphonic acid (AMPS) which possesses much industrial significance. The sulphonating agent used in the preparation reaction is typically oleum which is a mixture of sulphuric acid and sulphur trioxide. The concentration of sulphur trioxide in the oleum can vary, and it is known to influence the reaction. The water content in the reaction mixture is another factor that can influence the reaction. In particular, the presence of water can lead to side reactions, which can reduce the yield and purity of the resulting AMPS. Additionally, the reactor used in the reaction can be made of various materials, including metals and metal alloys, and the choice of material can also influence the corrosion resistance of the reactor, which can in turn influence the yield and purity of the resulting AMPS.

The preparation techniques available in the art exhibits challenges regarding water content leading to side reactions and formation of by products in high levels. In addition, the available AMPS preparation techniques also have disadvantages in aspects of poor yield and low purity along with additional washing steps consuming more amount of reagents/reactants such as acrylonitrile. Moreover, the presently available processes for the preparation of AMPS employ higher weight ratios of the reactant acrylonitrile and isobutene and still result in lower yields. In accordance with the above-mentioned points, the present disclosure provides a process for the preparation of AMPS wherein the sulphur trioxide is controlled by step-wise addition of the sulphonating reagent in multiple concentrations. The process disclosed in the present disclosure eliminates the problems associated with water content leading to side reactions, by usage of acrylonitrile substantially free of water and resulting in lowering the amount of by products in the final product. Furthermore, the present disclosure provides a continuous process using a first reactor and second reactor made of metal alloys specifically to maintain an inert atmosphere and result in lower reactivity of the reactants with the walls of the reactor so as to obtain the desired product in better yields and purity.

In an embodiment of the present disclosure, there is provided a process as disclosed herein, wherein the process involves the reaction of acrylonitrile with oleum in the presence of gaseous isobutene.

In an embodiment of the present disclosure, there is provided a process of preparation of 2-acrylamido-2-methylpropanesulphonic acid (AMPS), the process comprising: a. feeding a first reactor with acrylonitrile, isobutene and adding a first part of a sulphonating agent under stirring, followed by addition of a second part of the sulphonating agent; b. refeeding the first reactor with acrylonitrile, the sulphonating agent and isobutene, under stirring to obtain a mixture; c. allowing the mixture to overflow to a second reactor to obtain a slurry; and d. processing the slurry to obtain 2-acrylamido-2-methylpropanesulphonic acid, wherein acrylonitrile and isobutene are in a mole ratio range of 4:1 to 7:1.

In an embodiment of the present disclosure, there is provided a process of preparation of 2-acrylamido-2-methylpropanesulphonic acid (AMPS), the process comprising:
a. feeding a first reactor with acrylonitrile, isobutene and adding a first part of a sulphonating agent under stirring without residual water content, followed by addition of a second part of the sulphonating agent to obtain a mixture having no residual water content;
b. refeeding the first reactor with acrylonitrile, the sulphonating agent and isobutene, under stirring to obtain the mixture having no residual water content;
c. allowing the mixture to overflow to a second reactor to obtain a slurry; and
d. processing the slurry to obtain 2-acrylamido-2-methylpropanesulphonic acid, wherein acrylonitrile and isobutene are in a mole ratio range of 4:1 to 7:1.

In another embodiment of the present disclosure, acrylonitrile and isobutene are in a mole ratio range of 4.5:1 to 6.5:1. In another embodiment of the present disclosure, acrylonitrile and isobutene are in a mole ratio range of 5:1 to 6:1. In still another embodiment of the present disclosure, acrylonitrile and isobutene are in a mole ratio range of 5.5:1 to 6:1. In a further embodiment of the present disclosure, acrylonitrile and isobutene are in a mole ratio range of 5.5:1 to 5.8:1.

In an embodiment of the present disclosure, there is provided a process as disclosed herein, wherein the process is carried out in a continuous, batch, or semi-continuous mode. In another embodiment of the present disclosure, wherein feeding is carried out in a continuous, batch, or semi-continuous mode. In another embodiment of the present disclosure, wherein refeeding is carried out in a continuous, batch, or semi-continuous mode. In another embodiment of the present disclosure, the process (feeding and/or refeeding) is carried out in continuous mode. In one another embodiment of the present disclosure, the process (feeding and/or refeeding) is carried out in batch mode. In still another embodiment of the present disclosure, the process (feeding and/or refeeding) is carried out in semi-continuous mode.

In an embodiment of the present disclosure, there is provided a process as disclosed herein, wherein the sulphonating agent comprises sulphuric acid and sulphur trioxide. In another embodiment of the present disclosure, the sulphonating agent is added in two or three parts.

In an embodiment of the present disclosure, there is provided a process as disclosed herein, wherein the first part of the sulphonating agent is in an amount ranging from 20 to 40% (w/w) with respect to total amount of the sulphonating agent in step (a); and the second part of the sulphonating agent is in an amount ranging from 60 to 80% (w/w) with respect to total weight of the sulphonating agent in step (a). In another embodiment of the present disclosure, the first part of the sulphonating agent is in an amount ranging from 25 to 35% (w/w) with respect to total amount of the sulphonating agent of the first step; and the second part of the sulphonating agent is in an amount ranging from 65 to 75% (w/w) with respect to total weight of the sulphonating agent in step (a). In yet another embodiment of the present disclosure, the first part of the sulphonating agent is an amount ranging from 28 to 32% (w/w) with respect to total amount of the sulphonating agent of the first step; and the second part of the sulphonating agent is in an amount ranging from 68 to 72% (w/w) with respect to total weight of the sulphonating agent in step (a).

In an embodiment of the present disclosure, there is provided a process as disclosed herein, wherein the first part of the sulphonating agent have a sulphur trioxide content in the range of 20 to 30% (w/w) with respect to the total weight of the first part of the sulphonating agent, and the second part of the sulphonating agent have a sulphur trioxide content in the range of 1 to 5% (w/w) with respect to the total weight of the second part of the sulphonating agent. In another embodiment of the present disclosure, the first part of the sulphonating agent have a sulphur trioxide content in the range of 22 to 28% (w/w) with respect to the total weight of the first part of the sulphonating agent, and the second part of the sulphonating agent have a sulphur trioxide content in the range of 2 to 4% (w/w) with respect to the total weight of the second part of the sulphonating agent. In yet another embodiment of the present disclosure, the first part of the sulphonating agent have a sulphur trioxide content in the range of 22 to 25% (w/w) with respect to the total weight of the first part of the sulphonating agent, and the second part of the sulphonating agent have a sulphur trioxide content in the range of 2 to 3% (w/w) with respect to the total weight of the second part of the sulphonating agent.

In an embodiment of the present disclosure, there is provided a process as disclosed herein, wherein acrylonitrile, the sulphonating agent and isobutene in step (b) are added in same amount as added in step (a).

In an embodiment of the present disclosure, there is provided a process as disclosed herein, wherein the sulphonating agent in step (b) have sulphur trioxide in an amount ranging from 3 to 10% (w/w), with respect to total weight of the sulphonating agent of the step (b). In another embodiment of the present disclosure, the sulphonating agent in step (a) and in step (b) independently comprises sulphur trioxide in an amount ranging from 0.7% to 1% (w/w) with respect to the weight of acrylonitrile. In yet another embodiment of the present disclosure, the acrylonitrile has water content in a range of 0.1 to 0.5% (w/w).

In an embodiment of the present disclosure, there is provided a process as disclosed herein, wherein the acrylonitrile is anhydrous, with a water content of less than 0.43% (w/w) at the beginning of the process and less than 0.1% (w/w) by the end of the process. The water content of the acrylonitrile is confirmed through Karl Fischer (KF) equipment analysis.

In an embodiment of the present disclosure, there is provided a process as disclosed herein, wherein anhydrous acrylonitrile is used as one of the reactants. The term "anhydrous" as used herein refers to a substance that contains no water, negligible amount of water or has had all water removed. In an aspect of the present disclosure, anhydrous acrylonitrile refers to acrylonitrile that contains a specific water content in a range of 0.1 to 0.5% (w/w). This specific water content is selected to optimize the reaction conditions and to ensure a high yield and purity of 2-acrylamido-2-methylpropanesulphonic acid. The water content of the anhydrous acrylonitrile is confirmed through Karl Fischer (KF) equipment analysis. Karl Fischer titration is a classic titration method in analytical chemistry that uses coulometric or volumetric titration to determine trace amounts of water in a sample. In the context of the present disclosure, Karl Fischer titration is used to accurately measure the water content of the anhydrous acrylonitrile.

In an embodiment of the present disclosure, there is provided a process as disclosed herein, wherein the anhydrous acrylonitrile is fed into the reactor in a specific amount as described herein. The specific amount of anhydrous acrylonitrile is selected based on the desired yield and purity of 2-acrylamido-2-methylpropanesulphonic acid, the specific conditions of the reaction, and the specific properties of the reactants. For example, if a higher yield of 2-acrylamido-2-methylpropanesulphonic acid is desired, the amount of anhydrous acrylonitrile that is fed into the reactor is increased. Conversely, if a higher purity of 2-acrylamido-2-methylpropanesulphonic acid is desired, the amount of water (in anhydrous acrylonitrile) is reduced or controlled to minimize the byproducts and increase purity of 2-acrylamido-2-methylpropanesulphonic acid.

In an embodiment of the present disclosure, there is provided a process as disclosed herein, wherein the process is carried out in the absence of water content, and the amount of sulphur trioxide is maintained in a range of 0.7% to 1% (w/w) with respect to the quantity of acrylonitrile via the two-step addition of sulphonating agent oleum.

In an embodiment of the present disclosure, there is provided a process as disclosed herein, wherein the anhydrous acrylonitrile is cooled to a specific temperature in a range of 0 to 20°C prior to the addition of the sulphonating agent. In another embodiment of the present disclosure, wherein the anhydrous acrylonitrile is cooled to a specific temperature in a range of 10 to 20°C prior to the addition of the sulphonating agent. The specific temperature is selected to optimize the reaction conditions and to ensure a high yield and purity of 2-acrylamido-2-methylpropanesulphonic acid. The temperature of the anhydrous acrylonitrile is controlled using a suitable temperature control device, such as a heating or cooling jacket, a heat exchanger, or a thermostat.

In an embodiment of the present disclosure, there is provided a process as disclosed herein, wherein the first reactor is maintained at a temperature in a range of 35 to 50°C, prior to refeeding. In another embodiment of the present disclosure, the first reactor is maintained at a temperature in a range of 40 to 50°C, prior to refeeding. This temperature range is selected to optimize the reaction conditions and to ensure a high yield and purity of 2-acrylamido-2-methylpropanesulphonic acid. The temperature of the first reactor is controlled using a suitable temperature control device, such as a heating or cooling jacket, a heat exchanger, or a thermostat.

In an embodiment of the present disclosure, there is provided a process as disclosed herein, wherein in step (a) feeding the first reactor include acrylonitrile having a residence time in a range of 3 to 5 hours, isobutene having a residence time in a range of 15 to 21 hours, and the sulphonating agent having a residence time in a range of 27 to 37 hours. In another embodiment of the present disclosure, in step (a) feeding the first reactor include acrylonitrile having a residence time in a range of 3.6 to 4.8 hours, isobutene having a residence time in a range of 15 to 20.1 hours, and the sulphonating agent having a residence time in a range of 27.1 to 36.1 hours. The specific residence times for the acrylonitrile, isobutene, and the sulphonating agent is selected to optimize the reaction conditions and to ensure a high yield and purity of 2-acrylamido-2-methylpropanesulphonic acid.

In an embodiment of the present disclosure, there is provided a process as disclosed herein, wherein the first reactor is re-fed with acrylonitrile, the sulphonating agent, and isobutene in the same amount as in step (a) under stirring at a speed in a range of 100 to 700 rpm to ensure thorough mixing of the reactants. In another embodiment of the present disclosure, the stirring speed is in a range of 200 to 600 rpm. In yet another embodiment of the present disclosure, the stirring speed is in the range of 300 to 500 rpm. The specific stirring speed depends on the specific requirements of the process, including the desired yield and purity of 2-acrylamido-2-methylpropanesulphonic acid, the specific conditions of the reaction, and the specific properties of the reactants. In more embodiments of the present disclosure, the stirring speed is gradually increased during the addition of the first part of the sulphonating agent and isobutene. For example, the stirring speed is initially set at a lower value, such as 100 rpm, and then gradually increased to a higher value, such as 700 rpm, over a period of time. This gradual increase in stirring speed help to ensure a thorough mixing of the reactants and to facilitate the reaction between the acrylonitrile, the sulphonating agent, and the isobutene.

The stirring speed is selected to ensure a thorough mixing of the reactants and to facilitate the reaction between the acrylonitrile, the sulphonating agent, and the isobutene. The stirring is carried out using a suitable stirring device, such as an impeller, a paddle, or a turbine.

In an embodiment of the present disclosure, there is provided a process as disclosed herein, wherein the process is carried out for a period in a range of 1 to 24 hours and at a temperature in a range of 15 to 50°C. In another embodiment of the present disclosure, the process is carried out at a temperature in a range of 20 to 50°C. In yet another embodiment of the present disclosure, the process is carried out at a temperature in a range of 25 to 50°C. The specific duration and temperature range is selected to optimize the reaction conditions and to ensure a high yield and purity of 2-acrylamido-2-methylpropanesulphonic acid. The duration and temperature of the process is controlled using suitable control devices, such as a timer and a thermostat. The temperature of the process is controlled using a suitable temperature control device, such as a heating or cooling jacket, a heat exchanger, or a thermostat. In still another embodiment of the present disclosure, the temperature of the process is gradually increased during the addition of the first part of the sulphonating agent and isobutene. For example, the temperature is initially set at a lower value, such as 15°C, and then gradually increased to a higher value, such as 50°C, over a period of time. This gradual increase in temperature help to ensure a thorough mixing of the reactants and to facilitate complete reaction between the acrylonitrile, the sulphonating agent, and the isobutene.

In an embodiment of the present disclosure, there is provided a process as disclosed herein, wherein the step (b) involves refeeding the first reactor with acrylonitrile, the sulphonating agent and isobutene in an amount same as in step (a), under stirring to obtain a mixture; step (c) involves allowing the mixture to overflow to a second reactor to obtain a slurry; and step (d) involves processing the slurry to obtain 2-acrylamido-2-methylpropanesulphonic acid with a yield of at least 94% (w/w) and a purity of at least 96%, wherein the processing comprises filtration, distillation, centrifugation, drying, crystallizing, or combinations thereof.

In an embodiment of the present disclosure, there is provided a process as disclosed herein, wherein the processing comprise filtration, distillation, centrifugation, drying, crystallizing, or combinations thereof. In another embodiment of the present disclosure, the drying is carried out under vacuum at a temperature in a range of 60 to 80°C for a period in a range of 2 to 4 hours. In another embodiment of the present disclosure, the drying is carried out at a temperature in a range of 65 to 75°C under vacuum for a period in a range of 2.5 to 3.5 hours. In yet another embodiment of the present disclosure, the drying step involve removing the remaining solvent from the centrifuged slurry to obtain a dry product. In still another embodiment of the present disclosure, the drying step is carried out using a suitable drying device, such as a vacuum dryer, a tray dryer, a rotary dryer, or any other suitable type of dryer. In another embodiment of the present disclosure, drying is carried out under vacuum.

In an embodiment of the present disclosure, there is provided a process as disclosed herein, wherein the filtration step involves passing the slurry through a filter to separate the solid components from the liquid components. The filter is a mechanical filter, a membrane filter, a centrifugal filter, or any other suitable type of filter. The filtration step is carried out at a specific pressure and temperature to optimize the filtration efficiency.

In an embodiment of the present disclosure, there is provided a process as disclosed herein, wherein the distillation step involves heating the filtered slurry to evaporate the volatile components. The evaporated components then be condensed and collected. The distillation step is carried out at a specific pressure and temperature to optimize the distillation efficiency.

In an embodiment of the present disclosure, there is provided a process as disclosed herein, wherein the centrifugation step involves spinning the filtered and distilled slurry at a high speed to separate the solid components from the liquid components based on their densities. The centrifugation step is carried out at a specific speed and for a specific duration to optimize the centrifugation efficiency.

In an embodiment of the present disclosure, there is provided a process as disclosed herein, wherein processing involves crystallizing which comprises dissolving the crude product obtained by the process as disclosed herein in a crystallizing solvent, evaporating under vacuum followed by cooling the dissolved AMPS to form crystals of 2-acrylamido-2-methylpropanesulphonic acid. The crystallizing step is carried out at a specific temperature and for a specific duration to optimize the crystallization efficiency. The crystals of 2-acrylamido-2-methylpropanesulphonic acid then be collected and further processed as desired.

In an embodiment of the present disclosure, there is provided a process as disclosed herein, wherein the process is carried out in a reactor, which is made of a metal or metal alloy, such as nickel, chromium, molybdenum, or combinations thereof. In some aspects of the process, the first reactor and the second reactor are made of specific materials. The materials used for the first and second reactors are selected based on their resistance to corrosion, their thermal conductivity, their mechanical strength, and other relevant properties. In still another embodiment of the present disclosure, the first reactor is made of a specific metal alloy, such as Inconel 825. Inconel 825 is a nickel-ironchromium alloy with additions of molybdenum, copper and titanium. This alloy is known for its excellent resistance to both reducing and oxidizing acids, to stress-corrosion cracking, and to localized attack such as pitting and crevice corrosion. In one another embodiment of the present disclosure, the second reactor is made of a metal or metal alloy comprising nickel, chromium, molybdenum, or combinations thereof. In yet another embodiment of the present disclosure, the second reactor is made of a specific metal alloy, such as Hastelloy C-22. Hastelloy C-22 is a nickel-chromium-molybdenum-tungsten alloy with outstanding resistance to pitting, crevice corrosion and stress-corrosion cracking. This alloy is known for its excellent resistance to both oxidizing and reducing acid environments as well as those containing mixed acids. In further embodiments of the present disclosure, the first reactor and the second reactor are made of different materials. For example, the first reactor is made of Inconel 825, while the second reactor is made of Hastelloy C-22. The selection of different materials for the first and second reactors is based on the specific requirements of the process, including the desired yield and purity of 2-acrylamido-2-methylpropanesulphonic acid, the specific conditions of the reaction, and the specific properties of the reactants.

In an embodiment of the present disclosure, there is provided a process as disclosed herein, wherein the first and second reactor are independently equipped with a circulator arrangement to facilitate thorough mixing of the reactants. The circulator arrangement is configured to ensure a continuous flow of the reactants and the reaction mixtures throughout the process. The circulator arrangement includes a pump, a valve, a conduit, or combinations thereof. The pump is configured to pump the reactants and the reaction mixtures from one part of the process to another. The valve is configured to control the flow rate of the reactants and the reaction mixtures throughout the process. The conduit is configured to provide a flow path for the reactants and the reaction mixtures throughout the process. In yet another embodiment of the present disclosure, the circulator arrangement is used to facilitate the overflow of the second mixture from the first reactor to the second reactor. The circulator arrangement is configured to ensure a continuous flow of the second mixture from the first reactor to the second reactor. This helps to ensure a thorough mixing of the reactants and to facilitate the reaction between the acrylonitrile, the sulphonating agent, and the isobutene.

In an embodiment of the present disclosure, there is provided a process as disclosed herein, wherein following the refeeding of the first reactor, the mixture obtained in the first reactor is allowed to overflow into a second reactor to obtain a slurry. The overflow of the mixture into the second reactor is facilitated by a circulator arrangement. The circulator arrangement is configured to ensure a continuous flow of the second mixture from the first reactor to the second reactor. The circulator arrangement includes a pump, a valve, a conduit, or combinations thereof. The pump is configured to pump the second mixture from the first reactor to the second reactor. The valve is configured to control the flow rate of the second mixture from the first reactor to the second reactor. The conduit is configured to provide a flow path for the second mixture from the first reactor to the second reactor.

In an embodiment of the present disclosure, there is provided a process as disclosed herein, wherein the step (a) involves feeding a first reactor with acrylonitrile, isobutene and adding a first part of a sulphonating agent under stirring, followed by addition of a second part of the sulphonating agent. In another embodiment of the present disclosure, wherein the first reactor is made of a specific metal or metal alloy comprising nickel, chromium, molybdenum, or combinations thereof. In another embodiment of the present disclosure, the acrylonitrile used in step (a) is anhydrous, having a water content in a range of 0.1 to 0.5% (w/w). In yet another embodiment of the present disclosure, the acrylonitrile is cooled to a temperature in the range of 0 to 30°C prior to the addition of the sulphonating agent.

In an embodiment of the present disclosure, there is provided a process as disclosed herein, the process comprising: a. feeding a first reactor with acrylonitrile, isobutene, and adding a first part of a sulphonating agent under stirring, followed by addition of a second part of the sulphonating agent. In another embodiment of the present disclosure, the second part of the sulphonating agent have a sulphur trioxide content in the range of 1 to 5% (w/w) with respect to the total weight of the second part of the sulphonating agent. The addition of the second part of the sulphonating agent is carried out under stirring at a speed in a range of 100 to 700 rpm to ensure thorough mixing of the reactants.

In an embodiment of the present disclosure, there is provided a process as disclosed herein, the process comprising: a. feeding a first reactor with acrylonitrile, isobutene and adding a first part of a sulphonating agent under stirring at a speed in a range of 100 to 700 rpm, followed by addition of a second part of the sulphonating agent; b. refeeding the first reactor with acrylonitrile, the sulphonating agent and isobutene, under stirring to obtain a mixture; c. allowing the mixture to overflow to a second reactor to obtain a slurry; and d. processing the slurry to obtain 2-acrylamido-2-methylpropanesulphonic acid, wherein acrylonitrile and isobutene are in a mole ratio range of 4:1 to 7: 1. In another embodiment of the present disclosure, the stirring speed in a range of 100 to 700 rpm is selected to ensure a thorough mixing of the reactants and to facilitate the reaction between the acrylonitrile, the sulphonating agent, and the isobutene. The stirring is carried out using a suitable stirring device, such as an impeller, a paddle, or a turbine.

In an embodiment of the present disclosure, there is provided a process as disclosed herein, wherein isobutene is fed in a flow rate range of 0.003 L/min to 5 L/min. In another embodiment of the present disclosure, isobutene is fed in a flow rate range of 0.005 mL/min to 4.8 L/min. In yet another embodiment of the present disclosure, liquid isobutene is fed in a flow rate range of 0.01 to 0.02 L/min or gaseous isobutene is fed in a flow rate range of 2.5 to 4.9 L/min. In still another embodiment of the present disclosure, liquid isobutene is fed in a flow rate range of 0.015 to 0.018 L/min and gaseous isobutene is fed in a flow rate range of 2.4 to 4.8 L/min. In another embodiment of the present disclosure, liquid isobutene is fed in a flow rate range of 0.0165 to 0.0175 mL/min and gaseous isobutene is fed in a flow rate range of 2.35 to 4.7 L/min.

In an embodiment of the present disclosure, there is provided a process as disclosed herein, wherein the acrylonitrile is fed in a flow rate range of 65 to 75 mL/min. In another embodiment of the present disclosure, the acrylonitrile is fed in a flow rate range of 67 to 73 mL/min. In yet another embodiment of the present disclosure, the acrylonitrile is fed in a flow rate range of 69 to 72 mL/min. In still another embodiment of the present disclosure, acrylonitrile is fed in a flow rate of 70.93 mL/min.

In an embodiment of the present disclosure, there is provided a process as disclosed herein, wherein sulphonating agent is fed in a flow rate range of 5 to 15mL/min. In another embodiment of the present disclosure, sulphonating agent is fed in a flow rate range of 7 to 12 mL/min. In yet another embodiment of the present disclosure, sulphonating agent is fed in a flow rate range of 8 to 10 mL/min. In still another embodiment of the present disclosure, sulphonating agent is fed in a flow rate of 9.41 mL/min.

In an embodiment of the present disclosure, there is provided a process as disclosed herein, wherein the process is carried out in an inert atmosphere. In another embodiment of the present disclosure, the process is carried out in an inert atmosphere, wherein the inert atmosphere comprises an inert gas selected from nitrogen, helium (He), neon (Ne), argon (Ar), krypton (Kr), xenon (Xe), or radon (Rn).

In an embodiment of the present disclosure, there is provided a process as disclosed herein, wherein purity of 2-acrylamido-2-methylpropanesulphonic acid is at least 94%. In another embodiment of the present disclosure, the purity of 2-acrylamido-2-methylpropanesulphonic acid is at least 96%. In yet another embodiment of the present disclosure, purity of 2-acrylamido-2-methylpropanesulphonic acid is at least 97%. In still another embodiment of the present disclosure, wherein purity of 2-acrylamido-2-methylpropanesulphonic acid is at least 98%. In still another embodiment of the present disclosure, wherein purity of 2-acrylamido-2-methylpropanesulphonic acid is at least 99%. In one another embodiment of the present disclosure, purity of 2-acrylamido-2-methylpropanesulphonic acid is 99.76%.

In an embodiment of the present disclosure, there is provided a process as disclosed herein, wherein the first mixture comprises acrylonitrile, the sulphonating agent, and isobutene, along with the reaction products and by-products. In another embodiment of the present disclosure, the mixture is subjected to further processing steps to obtain 2-acrylamido-2-methylpropanesulphonic acid.

In an embodiment of the present disclosure, there is provided a process as disclosed herein, wherein the process results in obtaining by-products selected from acrylamide, tertiary butyl acrylamide, 2-methyl-2-propenyl-1-sulphonic acid, 2-methylidene-1,3-propylenedisulphonic acid, or combinations thereof. In another embodiment of the present disclosure, 2-methyl-2-propenyl-1-sulphonic acid is in an amount less than 3,000 mass ppm, and 2-methylidene-1,3-propylenedisulphonic acid is in an amount less than 8,000 mass ppm.

In an embodiment of the present disclosure, there is provided a process as disclosed herein, wherein yield of 2-acrylamido-2-methylpropanesulphonic acid is at least 94% (w/w). In another embodiment of the present disclosure, wherein yield of 2-acrylamido-2-methylpropanesulphonic acid is 94.7% (w/w). In another embodiment of the present disclosure, the processing of the slurry results in obtaining 2-acrylamido-2-methylpropanesulphonic acid with a yield of at least 94% (w/w) and a purity of at least 96%. In another embodiment of the present disclosure, the specific yield and purity of 2-acrylamido-2-methylpropanesulphonic acid depend on the specific conditions and parameters of the process, including the specific amounts and concentrations of the reactants, the specific conditions of the reaction, and the specific steps and conditions of the processing of the slurry.

In an embodiment of the present disclosure, there is provided a process as disclosed herein, wherein 2-acrylamido-2-methylpropanesulphonic acid is subjected to purification. In another embodiment of the present disclosure, after purification purity of 2-acrylamido-2-methylpropanesulphonic acid is at least 99%. In another embodiment of the present disclosure, after purification purity of 2-acrylamido-2-methylpropanesulphonic acid is at least 99.5%. In yet another embodiment of the present disclosure, 2-acrylamido-2-methylpropane-1-sulphonic acid is recovered in a weight range of 90 to 96 % (w/w). In one another embodiment of the present disclosure, yield of 2-acrylamido-2-methylpropane-1-sulphonic acid after purification is 86.4 % (w/w) with respect to isobutene. The weight of 2-acrylamido-2-methylpropanesulphonic acid is determined using suitable weighing devices, such as a balance, a scale, or a weighing pan. The weight of 2-acrylamido-2-methylpropanesulphonic acid is adjusted by varying the amounts of the reactants, the conditions of the reaction, and the steps and conditions of the processing of the slurry. In more embodiments of the present disclosure, the weight of 2-acrylamido-2-methylpropanesulphonic acid is adjusted by varying the amounts of acrylonitrile, the sulphonating agent, and isobutene that are fed into the reactor. For example, if a higher weight of 2-acrylamido-2-methylpropanesulphonic acid is desired, the amounts of acrylonitrile, the sulphonating agent, and isobutene that are fed into the reactor is increased. Conversely, if a lower weight of 2-acrylamido-2-methylpropanesulphonic acid is desired, the amounts of acrylonitrile, the sulphonating agent, and isobutene that are fed into the reactor is decreased. In yet another embodiment of the present disclosure, the weight of 2-acrylamido-2-methylpropanesulphonic acid is adjusted by varying the conditions of the reaction. For example, if a higher weight of 2-acrylamido-2-methylpropanesulphonic acid is desired, the temperature of the reaction is increased, the pressure of the reaction is decreased, or the duration of the reaction is extended. In still another embodiment of the present disclosure, the weight of 2-acrylamido-2-methylpropanesulphonic acid is adjusted by varying the steps and conditions of the processing of the slurry. For example, if a higher weight of 2-acrylamido-2-methylpropanesulphonic acid is desired, the filtration step is carried out at a higher pressure, the distillation step is carried out at a higher temperature, or the drying step is carried out for a longer duration.

In an embodiment of the present disclosure, there is provided a process as disclosed herein, wherein in step (c) the second reactor is configured to receive the overflow of the mixture from the first reactor. In another embodiment of the present disclosure, the second reactor is made of a specific metal or metal alloy comprising nickel, chromium, molybdenum, or combinations thereof. In still another embodiment of the present disclosure, the second reactor is equipped with a circulator arrangement to facilitate thorough mixing of the mixture. In yet another embodiment of the present disclosure, the second reactor is maintained at a specific temperature in a range of 35 to 50°C, to facilitate the reaction between the acrylonitrile, the sulphonating agent, and the isobutene in the mixture. In another embodiment of the present disclosure, wherein step (c) involves allowing the mixture to overflow to a second reactor to obtain a slurry; and step (d) involves processing the slurry to obtain 2-acrylamido-2-methylpropanesulphonic acid, the second reactor is stirred at a speed in a range of 100 to 700 rpm. In another embodiment of the present disclosure, the stirring speed is selected to ensure a thorough mixing of the second mixture and to facilitate the reaction between the acrylonitrile, the sulphonating agent, and the isobutene. In still another embodiment of the present disclosure, following the stirring of the second mixture in the second reactor, a slurry is obtained. In yet another embodiment of the present disclosure, the slurry comprises acrylonitrile, the sulphonating agent, and isobutene, along with the reaction products and by-products. The slurry is subjected to further processing steps to obtain 2-acrylamido-2-methylpropanesulphonic acid.

In an embodiment of the present disclosure, there is provided a process as disclosed herein, wherein the molar ratio of acrylonitrile and isobutene is maintained in a range of 1:4 to 1:7. This molar ratio range is selected to optimize the reaction conditions and to ensure a high yield and purity of 2-acrylamido-2-methylpropanesulphonic acid. The molar ratio of isobutene and acrylonitrile is controlled by adjusting the amounts of isobutene and acrylonitrile that are fed into the reactor. In some cases, the molar ratio of isobutene and acrylonitrile is adjusted by varying the flow rates of isobutene and acrylonitrile into the reactor. The flow rates of isobutene and acrylonitrile is controlled using suitable flow control devices, such as flow meters, valves, or pumps. In yet another embodiment of the present disclosure, the flow rate of isobutene is adjusted by varying the speed of the pump, the opening of the valve, or the setting of the flow meter. In still another embodiment of the present disclosure, the flow rate of isobutene is adjusted by varying the pressure of the isobutene gas. For example, a higher pressure results in a higher flow rate of isobutene, while a lower pressure results in a lower flow rate of isobutene. The pressure of isobutene gas is controlled using a suitable pressure control device, such as a pressure regulator, a pressure gauge, or a pressure sensor. In another embodiment of the present disclosure, the flow rate of isobutene is adjusted by varying the temperature of the isobutene gas. For example, a higher temperature results in a higher flow rate of isobutene, while a lower temperature results in a lower flow rate of isobutene. The temperature of isobutene gas is controlled using a suitable temperature control device, such as a heating or cooling jacket, a heat exchanger, or a thermostat.

In an embodiment of the present disclosure, there is provided a process as disclosed herein, wherein the molar ratio of acrylonitrile and isobutene are maintained within a range of 4:1 to 7:1. In another embodiment of the present disclosure, the specific molar ratio of isobutene and acrylonitrile depend on the specific requirements of the process, including the desired yield and purity of 2-acrylamido-2-methylpropanesulphonic acid, the specific conditions of the reaction, and the specific properties of the reactants. In other embodiments of the present disclosure, the molar ratio of isobutene and acrylonitrile is adjusted by varying the amounts of isobutene and acrylonitrile that are fed into the reactor. For example, if a higher yield of 2-acrylamido-2-methylpropanesulphonic acid is desired, the amount of isobutene that is fed into the reactor is increased, while the amount of acrylonitrile that is fed into the reactor is decreased. Conversely, if a higher purity of 2-acrylamido-2-methylpropanesulphonic acid is desired, the amount of acrylonitrile that is fed into the reactor is increased, while the amount of isobutene that is fed into the reactor is decreased.

In an embodiment of the present disclosure, there is provided a composition comprising: a) a) 2-acrylamido-2-methylpropanesulphonic acid; b) 2-methyl-2-propenyl-1-sulphonic acid; and c) 2-methylidene-1,3-propylenedisulphonic acid.

In an embodiment of the present disclosure, there is provided a composition as disclosed herein, wherein the composition comprises 2-methyl-2-propenyl-1-sulphonic acid is in an amount less than 3,000 mass ppm and 2-methylidene-1,3-propylenedisulphonic acid is in an amount less than 8,000 mass ppm.

A number of implementations have been described. Nevertheless, it will be understood that various modifications is made without departing from the spirit and scope of the disclosure. Accordingly, other implementations are within the scope of the claims.

### EXAMPLES

The disclosure will now be illustrated with working examples, which is intended to illustrate the working of disclosure and not intended to take restrictively to imply any limitations on the scope of the present disclosure. Unless defined otherwise, all technical and scientific terms used herein have the same meaning as commonly understood to one of ordinary skill in the art to which this disclosure belongs. Although methods and materials similar or equivalent to those described herein can be used in the practice of the disclosed methods, the exemplary methods, devices and materials are described herein. It is to be understood that this disclosure is not limited to particular methods, and experimental conditions described, as such methods and conditions may apply.

### EXAMPLE 1

### Preparation of 2-acrylamido-2-methylpropanesulphonic acid (AMPS) - Process 1

A first reactor made of Inconel 825 and a second reactor made of Hastelloy C 22 each having a capacity of 25 liters were ensured to be clean and dry (4-stage impeller, continuous stirred-tank reactor (CSTR) mode). The bottom valves of both reactors were closed, and nitrogen gas (inert atmosphere) was passed at 30 to 60 litre per hour (LPH) flow rate until the reaction was complete in both reactors.

Step (a): In a continuous process, the first reactor was fed with 6860g of fresh acrylonitrile having a water content of 0.43% w/w (as measured using Karl Fischer (KF) equipment analysis) using a peristaltic pump at a flow rate of 70.93 mL/min. The first reactor was then cooled to a temperature of 15°C and stirred at a stirring speed of 200 rpm which was slowly increased to 600 rpm. Subsequently, 650 g (31% with respect to total oleum added in first step) of oleum comprising **23%** (w/w) of sulphur trioxide (first part of a sulphonating agent) was added at a flow rate of 9.41 mL/min into the acrylonitrile under stirring, followed by addition of 1450 g (69% with respect to total oleum added in first step) of oleum comprising **2.38%** (w/w) of sulphur trioxide (second part of the sulphonating agent) at a flow rate of 9.41 mL/min for 120 minutes, in the presence of 1207 g of isobutene gas at a flow rate of 4 L/min for 120 minutes. The first reactor temperature was increased from 15°C to 45°C over a period of 1 hour and was maintained at a temperature in a range of 45°C(+/-5°C) where sulphur trioxide content was maintained in an amount of 0.77% (w/w).

Step (b): After 120 minutes, the same quantity of acrylonitrile (6860 g with water content of 0.43% (w/w)) at a flow rate of 70.93 mL/min, 2100g of oleum comprising 8.83% (w/w) of sulphur trioxide (sulphonating agent) at a flow rate of 9.41 mL/min, and 1210g of isobutene at a flow rate of 4 L/min were refed to the first reactor under stirring at a speed of 500 ± 50 rpm and maintaining at a temperature of 45°C (+/-5°C) using hot water circulation, to obtain a mixture, where the sulphur trioxide content was maintained in an amount of 0.78% (w/w).

Step (c): As the mixture reached the overflow line in the first reactor after a period of 210 minutes, it was then connected to the second reactor and the reaction was carried out for a period of 8 to 10 hours in first reactor. The mixture was then allowed to overflow to the second reactor to obtain a slurry.

Step (d): The slurry from the second reactor was then subjected to filtration under vacuum, and drying at a temperature of 70°C (+/-5°C) under vacuum in a rotavapor for 2 to 3 hours to obtain a crude cake of 2-acrylamido-2-methylpropanesulphonic acid. The yield of obtained crude AMPS was 94.7% (with respect to total weight of reacted isobutene) and a purity of 96.82%.

The obtained crude AMPS also comprised N-tertiary butyl acrylamide (N-TBA), 3000ppm of 2-methyl-2-propenyl-1-sulphonic acid (IBSA) and 8000 ppm of 2-methylidene-1,3-propylenedisulphonic acid (IBDSA). Figure 1 depicts the schematic representation of the first reactor and second reactor wherein the circulator arrangement of the reactors is depicted.

The obtained crude AMPS was then subjected to purification, wherein a 20 litre round bottom (RB) flask was set up on hot water bath with an agitator, a temperature controller, a thermo well, and a blanketing tube for passing nitrogen. The RB flask was charged with 1500 g of crude 2-acrylamido-2-methylpropanesulphonic acid and 7.5 litre of 10 % aq. acetic acid was added with a simultaneous stirring at 200 to 300 rpm. The mixture was heated at a temperature of 85°C to dissolve AMPS in the solution of acetic acid. The obtained mixture was filtered through G 1 sintered funnel using vacuum. The filtrate was collected in 20 litre rota flask and was subjected to distillation to distill out 85 % of aq. acetic acid at a temperature of 74 to 76 °C under a vacuum of 90 to 110 mbar. The resulting slurry was then stirred at a speed of 70 rpm under nitrogen atmosphere. The weight of the obtained slurry and distilled acetic acid were checked. To the obtained slurry, 375 g of fresh acetic acid (or acetone) was added and cooled to 30°C while stirring at 30 rpm at a rate of 5°C/0.5 hr. The resulting mixture was maintained for 1 hr at 30°C under agitation and further centrifuged. Centrifugation was carried out by feeding the slurry at 400 rpm, spinning at 1400 rpm for 20 min, washing using 1150 g of acetic acid or acetone followed by centrifuging at 300 rpm for 30 min, and again centrifuging at 1400 rpm for 30 min. The obtained residue was then dried at 90°C for 3 hrs under vacuum to obtain a solid. The weight of purified solid comprising pure AMPS was found to be 1325g (86.4% with respect to the total weight of reacted isobutene) and purity was found to be more than 99.5 % (w/w).

**Table 1**

| **S. No** | **Component in purified sample** | **Content** |
|---|---|---|
| 1 | AMPS (Purity %) | 99.76 |
| | Pure AMPS (Yield %) | |
| | | 86.4 (with respect to total weight of reacted isobutene) |
| 2 | Acrylonitrile (%) | ND |
| 3 | Acrylamide (ppm) | 74 |
| 4 | N-TBA (ppm) | 900 |
| 5 | IBSA (ppm) | ND |
| 6 | IBDSA (ppm) | 32 |
| 7 | AcOH (ppm) | 935 |
| 8 | Acrylic acid (ppm) | 133 |
| 9 | Meth acrylic acid (ppm) | 125 |
| 11 | Water (ppm) | 1800 |
| 12 | Melting point (°C) | 184.5 |
| 13 | Weight (g) | 1325 |

| | | |
|---|---|---|
| *ND- Less than 5 ppm | | |

The byproducts IBSA and IBDSA were found to be in minimal amounts in the purified sample. Yield of the obtained purified AMPS was found to be recovered in a weight of 91.2 % (w/w) (with respect to the total weight of the crude AMPS obtained).

The volume of the first and second reactors was 20.430 L each. The flow rates of acrylonitrile was 70.93mL/min, isobutylene was 4 mL/min, and oleum was 9.41 mL/min. Hence the residence time calculated for acrylonitrile was 288 min, isobutylene was 1206 min, and oleum was 2171 min.

### Characterization of the AMPS obtained by the Process 1

The below Table 2 shows the analysis results of crude AMPS obtained by the process 1 as explained above. The yield and purity of the AMPS product obtained are tabulated below.

**Table 2**

| S. No | Content | Process 1 |
|---|---|---|
| 1 | AMPS Purity % | 96.82 |
| 2 | AMPS Yield % | 94.7 |
| 3 | Acrylamide % | 0.36 |
| 4 | N-TBA % | 0.88 |
| 5 | IBSA % | 0.12 |
| 6 | IBDSA % | 0.31 |
| 7 | Melting Point °C | 177.8 |
| 8 | Water % | 0.9 |
| 9 | Weight of AMPS in g | 6665 |

It was observed that the Process 1 provided AMPS with enhanced purity and higher yield (~96.82 %;94.7), in varying moisture content of acrylonitrile and with distilled/recycled acrylonitrile.

For comparative purposes, AMPS was prepared by adding the sulphonating agent only once with a specific concentration.

Process **1a** was carried out as described herein, wherein oleum having 23% of sulphur trioxide was added once.

Step (a): The first reactor made of Inconel 825 material was fed with 6860 g of fresh acrylonitrile having a water content of 0.43% w/w (as measured using Karl Fischer (KF) equipment analysis) using a peristaltic pump. The first reactor was then cooled to a temperature of 15°C and stirred at a stirring speed of 200 rpm and slowly increased to 600 rpm. Subsequently, 2100 g of oleum comprising 23% (w/w) of sulphur trioxide was added into the acrylonitrile under stirring, for 120 minutes, in the presence of 1210g of isobutene gas at a flow rate of 16.94 mL/min for 120 minutes. The first reactor temperature was increased from 15°C to 45°C over a period of 1 hour and was maintained at a temperature in a range of 45°C(+/-5°C) to obtain a mixture.

Step (b): After 120 minutes, the same quantity of acrylonitrile (6860 g with water content of 0.43% (w/w)) at a flow rate of 70.93 mL/min, 2100g of oleum comprising 23% (w/w) of sulphur trioxide (sulphonating agent) at a flow rate of 9.41 mL/min, and 1210g of isobutene at a flow rate of 4 L/min were refed to the first reactor under stirring at a speed of 500 ± 50 rpm and maintaining at a temperature of 45°C (+/-5°C) using hot water circulation, to obtain a mixture, where the sulphur trioxide content was maintained in an amount of 0.78% (w/w).

Step (c): The mixture was overflowed to a second reactor after a period of about 210 minutes and was then stirred for a period of 8 to 10 hours to obtain a slurry.

Step (d): The slurry was then subjected to filtration, centrifugation and drying at 70°C (+/-5°C) under vacuum in a rotavapor for 2 to 3 hours to obtain a crude cake of 2-acrylamido-2-methylpropanesulphonic acid.

Similarly, processes **1b** and **1c** were carried out with 8.83% and 2.38%, of sulphur trioxide in oleum respectively.

The obtained yield and purity of AMPS for the processes **1** (process of present disclosure) and comparative processes **1a**, **1b** and **1c**, are shown in Table 2 below.

**Table 2**

| **S.No** | **Process** | **Concentration of SO₃ in Oleum in %** | **Yield %** | **Purity in %** |
|---|---|---|---|---|
| 1 | Process 1 | 23, 2.38 and 8.83 | 86.4 | 99.76 |
| 2 | Process 1a | 23 | 71.6 | 97.5 |
| 3 | Process 1b | 8.83 | 52.7 | 97.1 |
| 4 | Process 1c | 2.38 | 19.4 | 94.2 |

Thus, it could be observed that the process of the present disclosure employing three different concentrations of sulphonating agent in stepwise addition resulted in better yield and higher purity of AMPS.

Further, it was observed that there was significant corrosion of the first and second reactors when stainless steel reactors SS-316 and SS-316L were used. However, Inconel 825 and Hastelloy C22 reactors showed better results as the corrosion of the alloy based materials were found to be very low.

In addition, the reactors used in the present disclosure are advantageous over the glass lined reactors (GLRs). The major disadvantage of GLRs are limitation in agitation speed; and poor heat transfer leading to the requirement of pressurized cooling facilities, which are expensive. Further, if the GLR lining or coating peeled off, it is very difficult to paste it. Replacing the vessel is a time-consuming process and will severely affect the production.

### ADVANTAGES OF THE PRESENT INVENTION

The present disclosure provides a convenient and efficient process for the preparation of 2-acrylamido-2-methylpropanesulphonic acid (AMPS) via a continuous process without wastage of any reactants. The process of the present disclosure not only resulted in higher yield but also provided improved purity of AMPS product via the purification process as disclosed herein. The process disclosed herein also provides AMPS with minimum of byproducts IBSA and IBDSA. Further, the process as disclosed herein maintains the amount of sulphur trioxide in a range of 0.7 to 1% (w/w). Moreover, the process disclosed in the present disclosure does not require any additional acrylonitrile washing once the product AMPS is obtained. Furthermore, the process disclosed herein also provides AMPS without any residual water content thereby avoiding and minimizing side reactions that led to poor yield otherwise.

## Claims

1. A process for preparation of 2-acrylamido-2-methylpropanesulphonic acid, the process comprising:
a. feeding a first reactor with acrylonitrile, isobutene and adding a first part of a sulphonating agent under stirring, followed by addition of a second part of the sulphonating agent;
b. refeeding the first reactor with acrylonitrile, the sulphonating agent and isobutene under stirring to obtain a mixture;
c. allowing the mixture to overflow to a second reactor to obtain a slurry; and
d. processing the slurry to obtain 2-acrylamido-2-methylpropanesulphonic acid, wherein acrylonitrile and isobutene are in a mole ratio range of 4:1 to 7: 1.

2. The process as claimed in claim 1, wherein the process (feeding/refeeding) is carried out in a continuous, batch or semi-continuous mode.

3. The process as claimed in claim 1, wherein acrylonitrile, the sulphonating agent and isobutene in step (b) are added in same amount as added in step (a).

4. The process as claimed in claim 1, wherein the sulphonating agent comprises sulphuric acid and sulphur trioxide,
and the sulphonating agent comprises sulphuric acid with 1 to 30% (w/w) sulphur trioxide, with
respect to total amount of the sulphonating agent.

5. The process as claimed in claim 1, wherein the first part of the sulphonating agent is in an amount ranging from 20 to 40% (w/w) with respect to total amount of the sulphonating agent of step (a); and the second part of the sulphonating agent is in an amount ranging from 60 to 80% (w/w) with respect to total amount of the sulphonating agent of step (a).

6. The process as claimed in claim 1, wherein the first part of the sulphonating agent has sulphur trioxide in an amount ranging from 20 to 30% (w/w) with respect to total weight of the first part of the sulphonating agent; and the second part of the sulphonating agent has sulphur trioxide in an amount ranging from 1 to 5% (w/w) with respect to total weight of the second part of the sulphonating agent.

7. The process as claimed in claim 1, wherein the sulphonating agent in step (b) has sulphur trioxide in an amount ranging from 3 to 10% (w/w), with respect to total weight of the sulphonating agent of step (b).

8. The process as claimed in claim 1, wherein the sulphonating agent in step (a) and in step (b) independently comprises sulphur trioxide in an amount ranging from 0.7% to 1% (w/w) with respect to weight of acrylonitrile.

9. The process as claimed in claim 1, wherein acrylonitrile has water content in a range of 0.1 to 0.5% (w/w), and isobutene is fed in a flow rate range of 0.001 to 5 L/min.

10. The process as claimed in claim 1, wherein the first reactor is cooled to a temperature in a range of 0 to 20°C, prior to adding the first part of the sulphonating agent, and the first reactor is maintained at a temperature in a range of 35 to 50°C, prior to refeeding.

11. The process as claimed in claim 1, wherein the process is carried out for a period in a range of 1 to 24 hours, at a temperature in a range of 15 to 50°C, and in an inert atmosphere.

12. The process as claimed in claim 1, wherein processing comprises filtration, distillation, centrifugation, drying, crystallizing, or combinations thereof., and drying is carried out at a temperature in a range of 60 to 80°C under vacuum, for a period in a range of 2 to 4 hours.

13. The process as claimed in claim 1, wherein the first reactor and the second reactor are independently made of metal or metal alloy comprising nickel, chromium, molybdenum, or combinations thereof.

14. The process as claimed in claim 1, wherein the process comprises a circulator arrangement.

15. The process as claimed in claim 1, wherein purity of 2-acrylamido-2-methylpropanesulphonic acid is at least 95%, and yield of 2-acrylamido-2-methylpropanesulphonic acid is at least 85% (w/w).

16. The process as claimed in claim 1, wherein the process results in obtaining by-products selected from acrylamide, tertiary butyl acrylamide, 2-methyl-2-propenyl-1-sulphonic acid, 2-methylidene-1,3-propylenedisulphonic acid, or combinations thereof, and 2-methyl-2-propenyl-1-sulphonic acid is in an amount less than 3,000 mass ppm, and 2-methylidene-1,3-propylenedisulphonic acid is in an amount less than 8,000 mass ppm.

17. The process as claimed in claim 1, wherein 2-acrylamido-2-methylpropanesulphonic acid is subjected to purification, and the purity of 2-acrylamido-2-methylpropanesulphonic acid is at least 99%.

18. A composition comprising: a) 2-acrylamido-2-methylpropanesulphonic acid; b) 2-methyl-2-propenyl-1-sulphonic acid; and c) 2-methylidene-1,3-propylenedisulphonic acid, wherein the composition comprises 2-methyl-2-propenyl-1-sulphonic acid is in an amount less than 3,000 mass ppm, and 2-methylidene-1,3-propylenedisulphonic acid is in an amount less than 8,000 mass ppm.
